# EUROPEAN PATENT SPECIFICATION

(11) **EP 4 509 064 B1**
(45) Date of publication and mention of the grant of the patent: **30.09.2026**
(21) Application number: 24190378.0
(22) Date of filing: 23.07.2024
(51) Int. Cl.: A61B 17/06

(54) **MEDICAL MESH ASSEMBLY**
MEDIZINISCHE MASCHENANORDNUNG
ENSEMBLE MAILLE MÉDICALE

(30) Priority: 14.08.2023 US 202318449250; 08.04.2024 KR 20240047279
(43) Date of publication of application: 19.02.2025
(73) Proprietor: Yoon, Sang Joon, Gwangju-si, Gyeonggi-do (KR)
(72) Inventor: Yoon, Sang Joon, Gwangju-si, Gyeonggi-do (KR)
(74) Representative: BCKIP Part mbB

(56) References cited:
- EP-B1- 2 146 646
- KR-A- 20180 126 403
- KR-A- 20220 045 599
- KR-B1- 101 486 456
- US-A1- 2017 348 090

## Description

### BACKGROUND

### Technical Field of the Invention

The present invention relates to a medical instrument and, in more detail, to a mesh assembly for cosmetic procedures and plastic procedures.

### Description of the Related Art

Wrinkles are an important matter relating to aging and aging of the outward appearance may be more clearly recognized due to wrinkles. Accordingly, procedures of effectively removing wrinkles and recovering the elasticity of the skin are generally used in the field of skin care

Botox injection and filler injection are used as wrinkle removal procedures. Further, there are procedures of inserting a thread into the skin to lift up the skin and increase the elasticity. In the procedures using a thread, a thread is inserted into the skin using a cannula or a needle. A thread is fixed in the tissues inside the skin, thereby serving to reduce wrinkles and lift up the skin. Various types of threads are used in the procedures using threads and there is a need for a new type of skin insertion structure having increased fixing force and pulling force.

US 2017/0348090 A1 discloses a device for use in supporting a tissue in a patient's body, comprising a support member adapted to engage at least a portion of a tissue. The support member comprises a first end and a second end. The support member further comprises a plurality of support elements, and first and second suspension members, the first suspension member being coupled to the first end of the support member, the second suspension member being coupled to the second end of the support member. At least one of the first and second suspension members is configured to be secured to a location in the patient's body. The plurality of support elements is configured to distribute a load, from the tissue engaged by the support member, imposed on the support member. At least one of the first and second suspension members is configured to transmit a force through the support member.

KR 2022 0045599 A discloses a medical thread comprising a core and a mesh body. The mesh body is made of a porous structure surrounding the core, and is basically formed of a mesh structure taking a shape like a mesh network.

### SUMMARY OF THE INVENTION

The invention is defined by the appendend claims.

The present invention has been made in an effort to solve the problems of the related art described above and an objective of the present invention is to provide a mesh assembly having improved fixing force and pulling force for biological tissues and having an excellent effect of coming in close contact with skin tissues and maintaining and fixing the skin tissues.

According to the present invention, a mesh assembly in accordance with claim 1 is provided. A mesh member of the mesh assembly is configured in a cylindrical shape by a plurality of linear members crossing each other, so that a mesh assembly having improved fixing force and pulling force for biological tissues and having an excellent effect of coming in close contact with skin tissues and maintaining and fixing the skin tissues can be provided.

### BRIEF DESCRIPTION OF THE DRAWINGS

The above and other objectives, features and other advantages of the present invention will be more clearly understood from the following detailed description when taken in conjunction with the accompanying drawings, in which:
FIG. 1 is a view showing a portion of a mesh member 100 constituting a mesh assembly according to an embodiment not forming part of the claimed invention;
FIG. 2 is a view showing a portion of a mesh member constituting a mesh assembly according to another embodiment of the present invention;
FIG. 3 is a view showing a mesh assembly including a mesh member and a core 200 according to another embodiment not forming part of the claimed invention;
FIG. 4 is a view showing a mesh assembly including a mesh member and a core according to another embodiment of the present invention;
FIG. 5 is a view exemplifying the direction of protrusions formed on a linear member;
FIG. 6 is a view exemplifying the area of a hole formed in a mesh member;
FIG. 7 is a view exemplifying the gaps of protrusions formed on a linear member;
FIG. 8 is a view showing a portion of a mesh member constituting a mesh assembly according to another embodiment not forming part of the claimed invention;
FIGS. 9A and 9B are views showing various embodiments of a plurality of protrusions formed on a linear member;
FIG. 10 is a view exemplarily showing a portion of a mesh assembly according to the present invention;
FIGS. 11 and 12 are views exemplarily showing an insertion tube that can be applied to a mesh assembly according to the present invention;
FIGS. 13 and 14 are views exemplarily showing another insertion tube that can be applied to a mesh assembly according to the present invention; and
FIGS. 15 and 16 are views exemplarily showing another insertion tube 400C that can be applied to a mesh assembly 40 according to the present invention.

### DETAILED DESCRIPTION OF EXEMPLARY EMBODIMENTS OF THE INVENTION

The present invention may be modified in various ways and implemented by various exemplary embodiments, so that specific exemplary embodiments are shown in the drawings and will be described in detail herein. However, it is to be understood that the present invention is not limited to the specific exemplary embodiments. Similar reference numerals are assigned to similar components in the following description of drawings.

Unless defined otherwise, it is to be understood that all the terms used in the specification including technical and scientific terms have the same meanings as those that are understood by those who skilled in the art. It will be further understood that terms such as terms defined in common dictionaries should be interpreted as having a meaning that is consistent with their meaning in the context of this specification and the relevant art and will not be interpreted in an idealized or overly formal sense unless expressly so defined herein.

Hereinafter, exemplary embodiments of the present invention will be described in more detail with reference to the accompanying drawings.

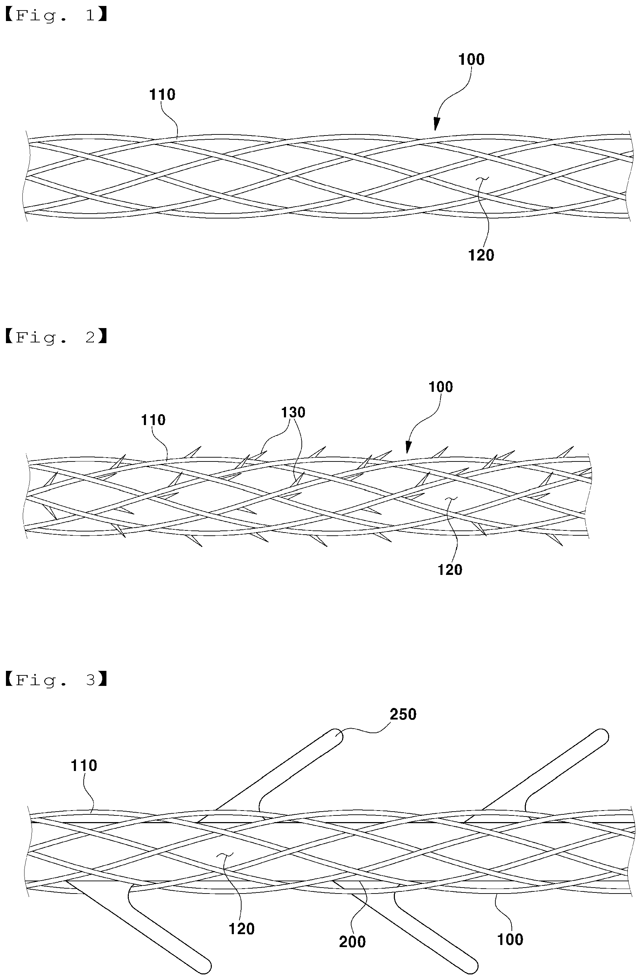

FIG. 1 is a view showing a portion of a mesh member 100 constituting a mesh assembly 10 according to an embodiment . Though not shown in FIG. 1, an insertion tube such as a cannula or a needle may be provided at the front end of the mesh assembly 10 and the mash member 100 may be coupled to or inserted in the insertion tube.

The mesh member 100 of the mesh assembly 10 is configured to be inserted into the skin tissue of a human body through an insertion tube such as a cannula. Referring to FIG. 1, the mesh member 100 of the mesh assembly 10 includes a plurality of linear members 110 and is formed in a cylindrical shape. Holes may be provided between the plurality of linear members 110.

The plurality of linear members 110 may spirally extend in the longitudinal direction of the mesh member 100. The plurality of linear members 110 may constitute a 3-dimensional cylindrical shape by being woven across each other.

The mesh member 100 may constitute a 3-dimensional shape similar to a cylinder by forming a passage with a predetermined diameter therein. In the embodiment shown in FIG. 1, the passage with a predetermined diameter formed in the mesh member 100 is empty. That is, another member is not disposed in the mesh member 100. However, in another embodiment to be described below, another member (a core, etc.) may be disposed in the mesh member 100.

The mesh member 100 may be made of a medial material that is used for implantation or insertion in a human body. For example, the mesh member 100 may be made of a nonabsorbable polymer, a biodegradable polymer, or the like that is not harmful to the human body and is biocompatible.

Further, the mesh member 100 may be made of a hydrolytic material. For example, the mesh member 100 may be made of a material containing polydioxanone (PDO). Further, the mesh member 100 may be made of a material containing one or more polymers of polylactic acid (PLA), poly-L-lactide (PLLA), polycaprolactone (PCL), polyhydroxyalkanoate (PHA), polyhydroxybutyrate (PHB), poly-D-lactide, and poly-DL-lactide (PDLLA).

In the embodiment shown in FIG. 1, the inside of the mesh member 100 of the mesh assembly 10 is empty. According to this configuration, when the mesh member 100 is inserted in a skin, skin tissues can easily permeate into the mesh member 100. Accordingly, combination of the mesh member 100 and skin tissues can be promoted.

FIG. 2 is a view showing a portion of a mesh member 100 constituting a mesh assembly 10 according to an embodiment of the present invention.

Referring to FIG. 2, the mesh member 100 of the mesh assembly 100 includes a plurality of linear members 110 and is formed in a cylindrical shape. Holes may be provided between the plurality of linear members 110.

The mesh member 100 of the mesh assembly 10 may be configured in a cylindrical shape forming a passage having a predetermined diameter therein. The passage in the mesh assembly 10 including the mesh member 100 is empty. The description of FIG. 1 can be referred to in relation to the mesh member 100.

The mesh member 100 includes a plurality of protrusions 130 protruding from the surface of the plurality of linear members 110. The protrusions 130 can fix the position of the mesh member 100 in a skin tissue or pull a skin by generating effective tension.

The plurality of protrusions 130 can generate tension by holding tissues in a skin. The plurality of protrusions 130 can serve to reduce or remove sagging or wrinkles of a skin. The plurality of protrusions 130 protrudes from the surfaces of the plurality of linear members 110 and may be arranged with predetermined gaps.

The plurality of protrusions 130 can provide a coupling force with skin tissues when pulling the skin in a procedure process. When the mesh member 100 is inserted in a skin by an insertion instrument and then the insertion instrument is removed, tension may be provided by a pulling force by a practitioner.

The plurality of protrusions 130 can serve to hold the position of the entire mesh member 100 pulled up by tension. Further, the plurality of protrusions 130 can prevent the mesh member 100 from moving down after a procedure. That is, the plurality of protrusions 130 is fixed to skin tissues in a procedure process, thereby being able to not only provide tension, but prevent sagging of the skin for a long time.

The plurality of protrusions 130 may be formed with regular gaps or different gaps on the plurality of linear members 110. A 3-dimensional cylindrical shape may be formed by spirally weaving the plurality of linear members 110 having the plurality of protrusions 130 across each other. The plurality of protrusions 130 may extend in random directions in the mesh member 100 having a 3-dimensional cylindrical shape. In another embodiment, a plurality of protrusions of the mesh member 100 may extend in a predetermined direction.

In the embodiment shown in FIG. 2, the inside of the mesh member 100 of the mesh assembly 10 is empty. According to this configuration, when the mesh member 100 is inserted in a skin, skin tissues can easily permeate into the mesh member 100. Accordingly, combination of the mesh member 100 and skin tissues can be promoted.

Further, in the embodiment of FIG. 2, a plurality of protrusions 130 is formed on a plurality of linear members 110 of the mesh member 100. According to this configuration, a fixing force and a pulling force of the mesh member 100 for skin tissues can be improved.

FIG. 3 is a view showing a mesh assembly 10 including a mesh member 100 and a core 200.

Referring to FIG. 3, a mesh assembly 10 may include a mesh member 100 and a core 200. The core 200 may include a cog protruding from the surface.

The mesh member 100 of the mesh assembly 100 includes a plurality of linear members 110 and is formed in a cylindrical shape. Holes may be provided between the plurality of linear members 110.

The mesh member 100 may be configured in a cylindrical shape forming a passage having a predetermined diameter therein. The core 200 extending in the longitudinal direction of the mesh member 100 may be disposed in the passage in the mesh assembly 10 including the mesh member 100.

The core 200 may be positioned in the mesh member 100. The core 200 may be formed with a diameter smaller than the diameter of the passage. The cross-section of the core 200 may be a circle. However, the cross-sectional shape of the core 200 is not limited to this embodiment and may be configured in various shapes such as an ellipse and a polygon.

The core 200 may include one or more cogs protruding and extending from the surface. The cog 250 may provide from the surface of the core 200 in an inclination direction toward a side. The cog 250 may be formed such that the end extends outside the mesh member 100. That is, the end of the cog 250 may protrude out of the mesh member 100 through a hole 120 between the plurality of linear members 110 included in the mesh member 100.

A plurality of cogs 250 may be formed on the upper portion and the lower portion of the core 200. That is, the plurality of cogs 250 may be formed alternately with gaps of 180 degrees on the outer circumferential surface of the core 200. However, the positions where the cogs 250 are formed are not limited to this embodiment, and, for example, the cogs 250 may be formed alternately with various gaps such as 90 degrees and 120 degrees on the outer circumferential surface of the core 200.

The core 200 and the mesh member 100 may be combined by thermal bonding (thermal fusion), an adhesive, or the like. That is, an end of the core 200 and an end of the mesh member 100 may be fused by heat or bonded by an adhesive, whereby the core 200 and the mesh member 100 can be coupled.

In the embodiment shown in FIG. 3, the core 200 is formed in the mesh member 100 of the mesh assembly 10 and cogs 250 protruding from the surface of the core 200 are formed on the core 200. According to this configuration, a fixing force and a pulling force of the mesh assembly 10 for skin tissues can be improved.

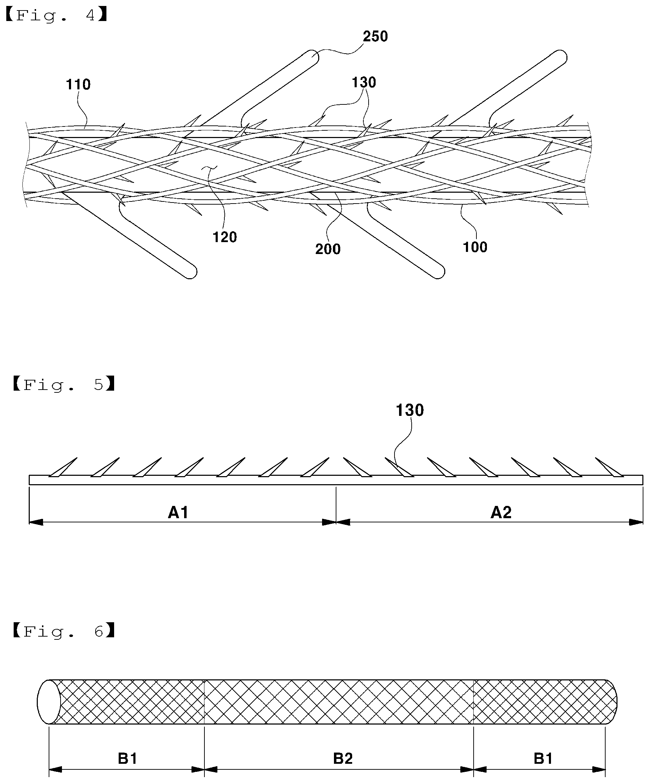

FIG. 4 is a view showing a mesh assembly 10 including a mesh member 100 and a core 200 according to another embodiment of the present invention.

Referring to FIG. 4, a mesh assembly 10 may include a mesh member 100 and a core 200. The mesh member 100 includes protrusions 130 protruding from the surface. The description about the protrusions 130 of the embodiment of FIG. 2 can be referred to for the mesh member 100 and the protrusions 130.

The core 200 may include cogs 250 protruding from the surface. The description about the core 200 and the cogs 250 of the embodiment of FIG. 3 can be referred to for the core 200 and the cogs 250.

In the embodiment shown in FIG. 4, the core 200 is formed in the mesh member 100 of the mesh assembly 10, the cogs 250 protruding from the surface of the core 200 are formed on the core 200, and protrusions 130 protruding from the surface of the mesh member 100 of the mesh assembly 10 are formed on the mesh member 100. According to this configuration, a fixing force and a pulling force of the mesh assembly 10 for skin tissues can be further improved.

FIG. 5 is a view exemplifying directions of the protrusions 130 formed on a linear member 110. One linear member 110 having a plurality of protrusions 130 is for manufacturing a mesh member 100. A mesh member 100 having a 3-dimensional cylindrical shape can be manufactured by weaving several pieces of the linear member 110 shown in FIG. 5 alternately across each other.

Referring to FIG. 5, the linear member 110 includes a plurality of protrusions 130 protruding from the surface. The linear member 110 includes a first section A1 and a second section A2 in which the extension directions of the protrusions are different.

The extension direction of the plurality of protrusions disposed in the first section A1 of the linear member 110 is different from the extension direction of the plurality of protrusions disposed in second section A2 of the linear member 110.

According to the present invention, as shown in FIG. 5, the extension direction of the plurality of protrusions 130 disposed in the first section A1 of the linear member 110 faces the extension direction of the plurality of protrusions disposed in second section A2 of the linear member 110. When a mesh member 100 having a cylindrical shape is manufactured by crossing several pieces of the linear member 110 in this embodiment across each other, the extension direction of the plurality of protrusions 130 disposed in the first section A1 may entirely face the extension direction of the plurality of protrusions disposed in second section A2.

As in the embodiment shown in FIG. 5, since the plurality of protrusions 130 disposed in the first section A1 faces the plurality of protrusions 130 disposed in second section A2, the fixing force and the pulling force of the mesh assembly 10 can be further improved.

FIG. 6 is a view exemplifying the area of the holes 120 formed on the mesh member 100. In an embodiment, the mesh member 110 may include a plurality of sections B1 and B2 in which the areas of holes 120 are different.

Referring to FIG. 6, the mesh member 100 may include a center section B2 and edge sections B1. In an embodiment, the area of the plurality of holes in the center section B2 of the mesh member 100 may be made larger than the area of the plurality of holes in the edge sections B1 of the mesh member 100. However, the present invention is not limited to the embodiment of FIG. 6, and, in another embodiment, the area of the plurality of holes in the center section B2 of the mesh member 100 may be made smaller than the area of the plurality of holes in the edge sections B1 of the mesh member 100.

Further, though not shown in FIG. 6, protrusions 130 protruding from the surface of the mesh member 100 may be formed on the mesh member 100 and the area of the holes may depend on the number and the gap of the protrusions 130 formed on the mesh member 100.

As in the embodiment shown in FIG. 6, the mesh member 100 is configured to include a plurality of sections B1 and B2 in which the areas of holes 120 are different, whereby it is possible to diversify the fixing force and the pulling force for skin tissues in the plurality of sections of the mesh member 100.

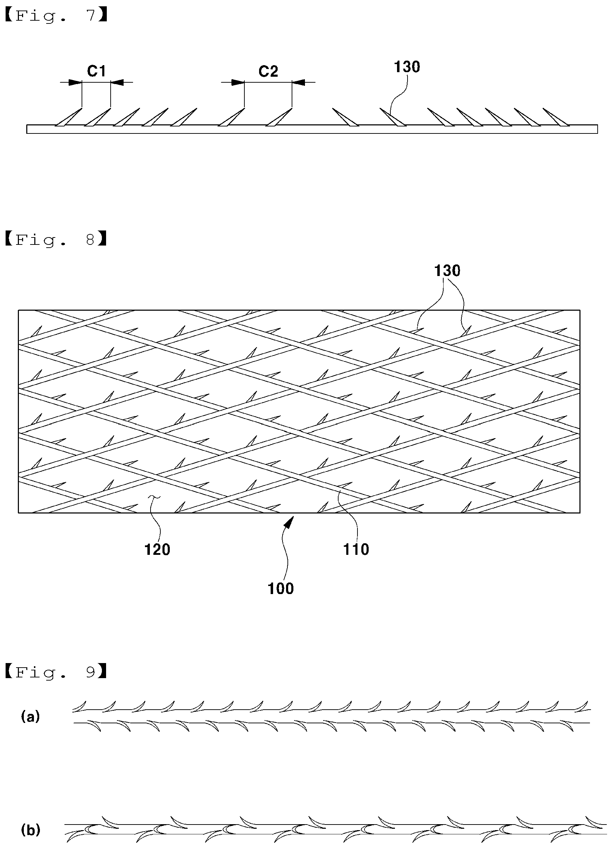

FIG. 7 is a view exemplifying the gaps of the protrusions 130 formed on a linear member 110.

Referring to FIG. 7, the linear member 110 includes a plurality of protrusions 130 protruding from the surface. In an embodiment, the linear member 110 may be configured such that the gap C1 of a plurality of protrusions 130 disposed at the edge portions of the linear member 110 may be made smaller than the gap C2 of a plurality of protrusions 130 disposed at the center portion of the linear member 110. When a mesh member 100 having a cylindrical shape is manufactured by alternately crossing several pieces of the linear member 110 of this embodiment, the gap C1 of the plurality of protrusions 130 disposed at the edge portions of the mesh member 100 is made smaller than the gap C2 of the plurality of protrusions 130 disposed at the center portion of the mesh member 100.

When the gaps of the protrusions 130 are the same, the fixing force and the pulling force for skin tissues may be relatively smaller at both end portions than the center portion of the mesh member 100. According to the embodiment shown in FIG. 7, the gap of the plurality of protrusions 130 at the edge portions is made smaller than the gap of the plurality of protrusions 130 at the center portion, and accordingly, it is possible to further improve the fixing force and the pulling force for skin tissues at both end portions of the mesh member 100.

In an embodiment, the hole area of meshes in the section of the mesh member 100 in which the gap of a plurality of protrusions 130 is small may be made larger than the hole area of meshes in the section of the mesh member 100 in which the gap of a plurality of protrusions 130 is large. In the section in which the gap of a plurality of protrusions 130 is small, the plurality of protrusions 130 may interfere with each other. When the hole area of meshes is made larger in the section in which the gap of a plurality of protrusions 130 is small, it is possible to reduce the influence by interference between the plurality of protrusions 130.

FIG. 8 is a view showing a portion of a mesh member 100 constituting a mesh assembly 10 according to another embodiment, which is not part of the claimed invention.

Referring to FIG. 8, the mesh member 100 of the mesh assembly 100 includes a plurality of linear members 110 crossing each other. The mesh member 100 may be configured in a plane shape having a predetermined area. The plurality of linear members 110 may include protrusions 130 protruding from the surface.

Unless contradicted as description about the mesh member 100, the linear members 110, and the protrusions 130 according to the embodiment of FIG. 8, the description about the mesh member 100, the linear members 110, and the protrusions 130 shown in FIGS. 1 to 7 can be referred to. The description about the extension direction of the protrusions 130 shown in FIG. 5, the hole area of the mesh member 100 shown in FIG. 6, and the gap of the protrusions 130 shown in FIG. 7 can be applied to the mesh member 100, the linear members 110, and the protrusions 130 according to the embodiment of FIG. 8.

FIG. 9A and 9B are views showing various embodiments of a plurality of protrusions 130 formed on a linear member 110.

FIG. 9A exemplifies a plurality of protrusions 130 arranged with gaps of 180 degrees in the circumferential direction of the linear member 110. Referring to FIG. 9A, a plurality of protrusions 130 may be longitudinally arranged. The plurality of protrusions 130 may be arranged alternately with gaps of 180 degrees in the circumferential direction.

FIG. 9B exemplifies a plurality of protrusions 130 arranged with gaps of 90 degrees in the circumferential direction of the linear member 110. Referring to FIG. 9B, a plurality of protrusions 130 may be longitudinally arranged. The plurality of protrusions 130 may be arranged alternately with gaps of 90 degrees in the circumferential direction.

Assuming that a protrusion 131 disposed on the lower portion of the linear member 110 is a first protrusion, a second protrusion 132 may be formed ahead with a gap of 90 degrees in the circumferential direction with respect to the first protrusion. A third protrusion 133 may be formed over with a gap of 90 degrees with respect to the second protrusion 132. A fourth protrusion is disposed behind and is not shown in the figures, but may be disposed behind with a gap of 90 degrees with respect to the third protrusion 133.

The shapes of the protrusions 130 shown in FIG. 2, FIG. 5, FIG. 9A, and FIG. 9B only exemplify the shape of protrusions 130 of linear members 110 according to the present invention and the present invention is not limited to this example.

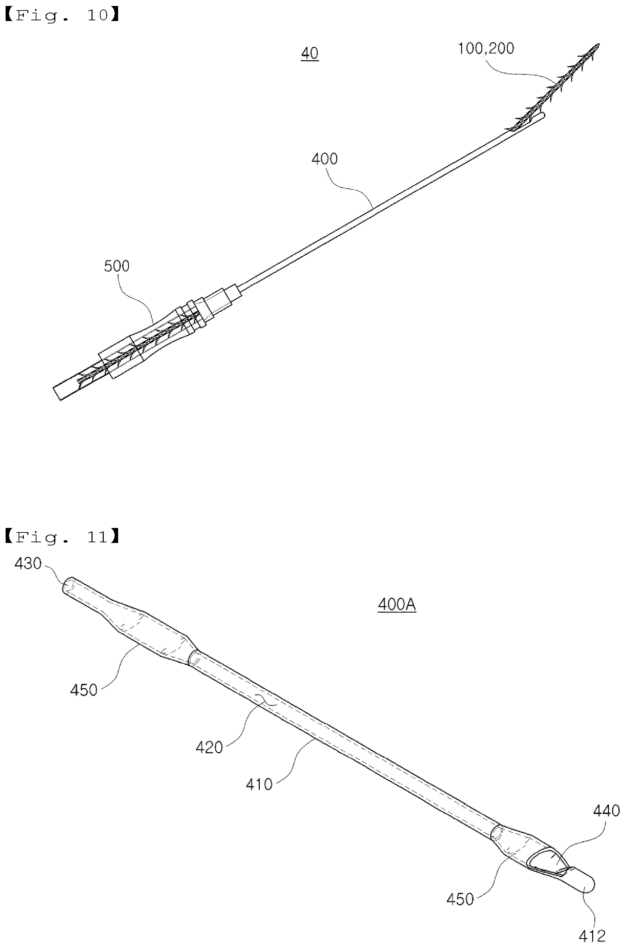

FIG. 10 is a view exemplarily showing a portion of a mesh assembly 40 according to the present invention.

Referring to FIG. 10, the mesh assembly 40 may further include an insertion tube 400 in the mesh assembly 10 relating to the embodiment described above. That is, the mesh assembly 40 may include a mesh member 100, a core 200, and an insertion tube 400. The mesh assembly 40 according to the present invention is not limited to the embodiment shown in FIG. 10 and may include a mesh member 100 and an insertion tube 400 without a core 200.

Assuming the extension direction of the core 200 is a first direction, the insertion tube 400 may extend in the first direction. The insertion tube 400 may include an internal channel space 420. In the present invention, the insertion tube 400 includes a cannula, Stewart needles, skin needles, catheter needles, injection needles, etc., and is a term including all kinds of medical instruments having a channel space 420 therein.

The channel space 420 may extend from an opening 430 positioned at the rear end of the insertion tube 400 to an opening 440 positioned at the front end of the insertion tube 400. As shown in FIG. 10, the mesh member 100 and the core 200 can be inserted into the channel space 420 of the insertion tube 400. The mesh member 100 and the core 200 is put into the opening 430 at the rear end of the channel space 420 and may be inserted to the opening 440 at the front end or beyond the opening 440 to pass through the opening 440. A connector 500 may be provided at the rear end of the insertion tube 400 for the convenience of use.

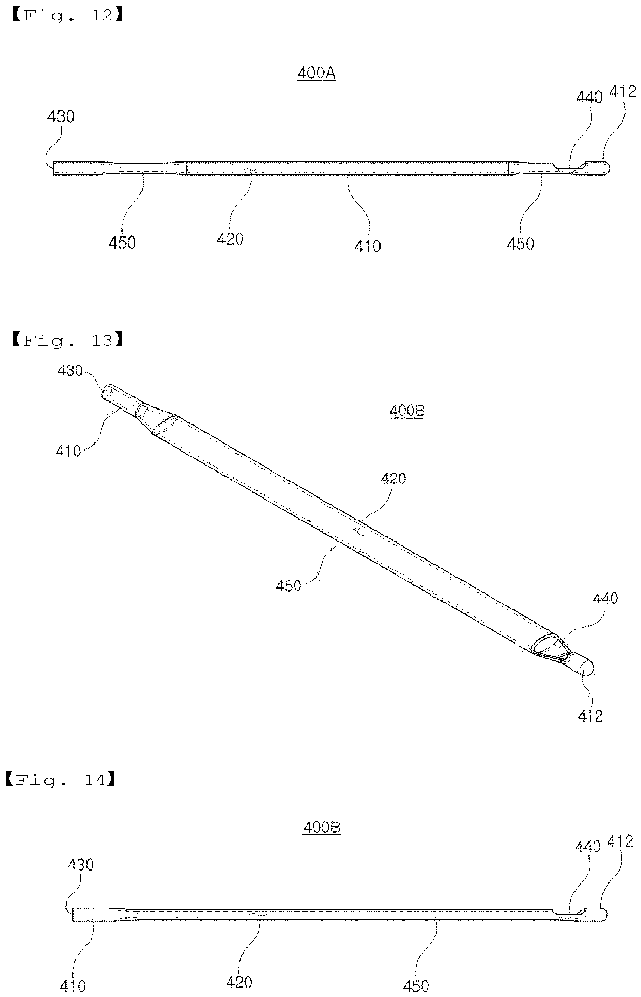

FIGS. 11 and 12 are views exemplarily showing an insertion tube 400A that can be applied to the mesh assembly 40 according to the present invention.

According to an embodiment of the present invention, the insertion tube 400A may include a crushed portion 450. In this case, the term 'crushed portion 450' is only for describing the shape of the insertion tube 400A and is not limited to being formed by compressing a main body 410 of the insertion tube 400A.

Assuming that the extension direction of the insertion tube 420 is a first direction, a direction perpendicular to the first direction is a second direction, and a direction perpendicular to both of the first direction and the second direction is a third direction, the main body 410 of the insertion tube 400 is crushed in the second direction at the crushed portion 450, whereby the cross-section of the crushed portion 450 may have a reduced width in the second direction. The crushed portion 450 may have an increased width in the third direction.

In the mesh assembly 40 relating to an embodiment of the present invention, since cogs 250 protrude from the core 200, a cross-section having a cog 250 of the core 200 is larger in width in a specific direction (i.e., the third direction) than another direction (i.e., the second direction). If the channel space 420 of the insertion tube 400 maintains a circular cross-section and has an increased inner diameter, and accordingly, when the mesh member 100 and the core 200 are accommodated therein, there is a possibility that the diameter of the entire insertion tube 400 becomes excessively large. In this case, there is a possibility that a hole having an increased size is formed at a procedure part of a patient and accordingly a scar is left.

When the crushed portion 450 is formed at the insertion tube 400, as in an embodiment of the present invention, the cross-section of the channel space 420 has a reduced width in the second direction and an increased with in the third direction. This insertion tube 400 is suitable for accommodating a core 200 having a small width in the second direction and a large width in the third direction. In an embodiment of the present invention, it is possible to substantially form the crushed portion 450 by compressing a portion of a cannula of a basic shape, for example, a portion of a cannula of 18 gauges.

In the example shown in FIGS. 11 and 12, a plurality of crushed portions 450 is formed at the insertion tube 400. The position and the number of the crushed portion 450 may be changed, if necessary. The front end 412 of the insertion tube 400 is rounded, so it may have a smooth curved surface without a sharp corner. The front opening 440 of the channel space 420 may be formed close to the front end 412.

FIGS. 13 and 14 are views exemplarily showing another insertion tube 400B that can be applied to the mesh assembly 40 according to the present invention.

In the insertion tube 400B shown in FIGS. 13 and 14, a crushed portion 450 is formed at a most part of the main body 410 of the insertion tube 400 and extends to the front opening 440. In the insertion tube 400B shown in FIGS. 13 and 14, the rear end of the main body 410 of the insertion tube 400 maintains a non-crushed state, but, in another embodiment, the crushed portion 450 may be formed throughout the entire main body 410 of the insertion tube 400 including the rear end of the main body 410.

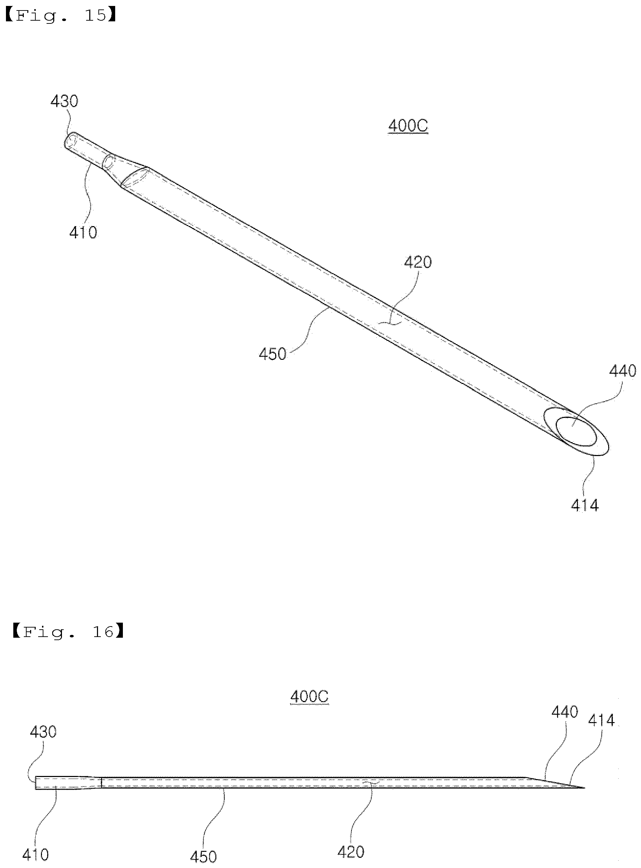

FIGS. 15 and 16 are views exemplarily showing another insertion tube 400C that can be applied to the mesh assembly 40 according to the present invention. In the insertion tube 400C shown in FIGS. 15 and 16, the front end 414 of the insertion tube 400 is formed in a shape having a cut surface and the front opening 440 of the channel space 420 is formed at the cut surface of the front end 414. This structure may have the advantage that the crushed portion 450 can extend to the front end 414.

## Claims

1. A mesh assembly (10) that is inserted in skin tissues of a human body, the mesh assembly (10) comprising a mesh member (100) having a plurality of linear members (110) crossing each other,
wherein the mesh member (100) includes a plurality of protrusions (130) protruding from the surface of the plurality of linear members (110), the mesh member (100) being formed in a cylindrical shape,
**characterized in that**,
an extension direction of the plurality of protrusions (130) in a first section (A1) of the linear member (110) faces an extension direction of the plurality of protrusions (130) in a second section (A2) of the linear member (110).

2. The mesh assembly (10) of claim 1, wherein the mesh member (100) is configured in the cylindrical shape forming a passage having a predetermined diameter therein,
the mesh assembly (10) further includes a core (200) positioned in the passage of the mesh member (100) and extending in a longitudinal direction of the mesh member (100), and
the core (200) includes a cog (250) protruding from a surface thereof.

3. The mesh assembly (10) of claim 1 or 2, wherein the protrusions (130) protrude from an outer circumferential surface of the linear members (110).

4. The mesh assembly (10) of any one of claims 1 to 3, wherein gaps (C1) of a plurality of protrusions (130) disposed at an edge portion of the linear member (110) are made smaller than gaps (C2) of a plurality of protrusions (130) disposed at a center portion of the linear member (110).

5. The mesh assembly (10) of any one of claims 1 to 4, wherein gaps (C1) of a plurality of protrusions (130) disposed at an edge portion of the linear member (110) are made smaller than gaps (C2) of a plurality of protrusions (130) disposed at a center portion of the linear member (110), and
areas of a plurality of holes at an edge portion (B1) of the mesh member (100) that corresponds to the edge portion of the linear member (110) are made larger than areas of a plurality of holes at a center portion (B2) of the mesh member (100) that corresponds to the center portion of the linear member (110).

6. The mesh assembly (10) of any one of claims 2 to 5, insofar as dependent upon claim 2, wherein ends of the mesh member (100) and the core (200) are coupled by thermal fusion or bonding.

7. The mesh assembly (10, 40) of any one of claims 1 to 6, further comprising an insertion tube (400, 400A, 400B, 400C) extending in a first direction that is a longitudinal direction, having a channel space (420) formed therein, and configured to accommodate the mesh member (100) in the channel space (420).

8. The mesh assembly (10, 40) of claim 7, wherein the insertion tube (400, 400A, 400B, 400C) includes a portion (450) crushed in a second direction perpendicular to the first direction, and a cross-section of the channel space (420) has a reduced width in the second direction at the crushed portion (450) and has an increased width in a third direction perpendicular to the first direction and the second direction at the crushed portion (450).

## Patentansprüche

1. Netzanordnung (10), die in Hautgewebe eines menschlichen Körpers eingeführt wird, wobei die Netzanordnung (10) ein Netzelement (100) umfasst, das eine Mehrzahl sich kreuzender Linearelemente (110) aufweist,
wobei das Netzelement (100) eine Mehrzahl von Vorsprüngen (130) umfasst, die von der Oberfläche der Mehrzahl von Linearelementen (110) vorstehen, wobei das Netzelement (100) in einer zylindrischen Form ausgebildet ist,
**dadurch gekennzeichnet, dass**
eine Erstreckungsrichtung der Mehrzahl von Vorsprüngen (130) in einem ersten Abschnitt (A1) des Linearelements (110) einer Erstreckungsrichtung der Mehrzahl von Vorsprüngen (130) in einem zweiten Abschnitt (A2) des Linearelements (110) zugewandt ist.

2. Netzanordnung (10) nach Anspruch 1, wobei das Netzelement (100) in der zylindrischen Form ausgebildet ist und darin einen Durchgang mit einem vorgegebenen Durchmesser bildet,
wobei die Netzanordnung (10) ferner einen Kern (200) umfasst, der in dem Durchgang des Netzelements (100) positioniert ist und sich in einer Längsrichtung des Netzelements (100) erstreckt, und
wobei der Kern (200) einen von einer Oberfläche desselben vorstehenden Widerhaken (250) umfasst.

3. Netzanordnung (10) nach Anspruch 1 oder 2, wobei die Vorsprünge (130) von einer Außenumfangsfläche der Linearelemente (110) vorstehen.

4. Netzanordnung (10) nach einem der Ansprüche 1 bis 3, wobei Abstände (C1) einer Mehrzahl von Vorsprüngen (130), die an einem Randbereich des Linearelements (110) angeordnet sind, kleiner ausgebildet sind als Abstände (C2) einer Mehrzahl von Vorsprüngen (130), die an einem Mittelbereich des Linearelements (110) angeordnet sind.

5. Netzanordnung (10) nach einem der Ansprüche 1 bis 4, wobei Abstände (C1) einer Mehrzahl von Vorsprüngen (130), die an einem Randbereich des Linearelements (110) angeordnet sind, kleiner ausgebildet sind als Abstände (C2) einer Mehrzahl von Vorsprüngen (130), die an einem Mittelbereich des Linearelements (110) angeordnet sind, und
wobei Flächen einer Mehrzahl von Öffnungen in einem Randabschnitt (B1) des Netzelements (100), der dem Randbereich des Linearelements (110) entspricht, größer ausgebildet sind als Flächen einer Mehrzahl von Öffnungen in einem Mittelabschnitt (B2) des Netzelements (100), der dem Mittelbereich des Linearelements (110) entspricht.

6. Netzanordnung (10) nach einem der Ansprüche 2 bis 5, soweit der betreffende Anspruch von Anspruch 2 abhängt, wobei Enden des Netzelements (100) und des Kerns (200) durch thermisches Verschmelzen oder Verbinden miteinander gekoppelt sind.

7. Netzanordnung (10, 40) nach einem der Ansprüche 1 bis 6, ferner umfassend ein Einführrohr (400, 400A, 400B, 400C), das sich in einer ersten Richtung, die eine Längsrichtung ist, erstreckt, einen darin ausgebildeten Kanalraum (420) aufweist und dazu ausgebildet ist, das Netzelement (100) in dem Kanalraum (420) aufzunehmen.

8. Netzanordnung (10, 40) nach Anspruch 7, wobei das Einführrohr (400, 400A, 400B, 400C) einen in einer zweiten Richtung, die senkrecht zu der ersten Richtung ist, zusammengedrückten Abschnitt (450) umfasst und ein Querschnitt des Kanalraums (420) an dem zusammengedrückten Abschnitt (450) in der zweiten Richtung eine verringerte Breite und in einer dritten Richtung, die senkrecht zu der ersten Richtung und der zweiten Richtung ist, an dem zusammengedrückten Abschnitt (450) eine vergrößerte Breite aufweist.

## Revendications

1. Ensemble de maille (10), inséré dans des tissus cutanés d'un corps humain, ledit ensemble de maille (10) comprenant un élément de maille (100) présentant une pluralité d'éléments linéaires (110) croisés entre eux,
où l'élément de maille (100) comprend une pluralité de saillies (130) s'étendant depuis la surface de la pluralité d'éléments linéaires (110), l'élément de maille (100) se présentant sous une forme cylindrique,
**caractérisé en ce que**
la direction d'extension de la pluralité de saillies (130) dans une première section (A1) de l'élément linéaire (110) est opposée à la direction d'extension de la pluralité de saillies (130) dans une deuxième section (A2) de l'élément linéaire (110).

2. Ensemble de maille (100) selon la revendication 1, où l'élément de maille (100) est prévu sous une forme cylindrique formant un passage ayant un diamètre intérieur prédéterminé,
l'ensemble de maille (10) comprend en outre un noyau (200) disposé dans le passage de l'élément de maille (100) et s'étendant dans le sens de la longueur de l'élément de maille (100), et
le noyau (200) comprend une dent (250) faisant saillie d'une de ses surfaces.

3. Ensemble de maille (10) selon la revendication 1 ou la revendication 2, où les saillies (130) s'étendent depuis une surface périphérique extérieure des éléments linéaires.

4. Ensemble de maille (10) selon l'une des revendications 1 à 3,
où les espacements (C1) d'une pluralité de saillies (130) disposées dans une partie de bord de l'élément linéaire (110) sont rendus plus petits que les espacements (C2) d'une pluralité de saillies (130) disposées dans une partie centrale de l'élément linéaire (110).

5. Ensemble de maille (10) selon l'une des revendications 1 à 4, où les espacements (C1) d'une pluralité de saillies (130) disposées dans une partie de bord de l'élément linéaire (110) sont rendus plus petits que les espacements (C2) d'une pluralité de saillies (130) disposées dans une partie centrale de l'élément linéaire (110), et
la surface d'une pluralité de trous dans une partie de bord (B1) de l'élément de maille (100) qui correspond à la partie de bord de l'élément linéaire (110) est agrandie par rapport à la surface d'une pluralité de trous dans une partie centrale (B2) de l'élément de maille (100) qui correspond à la partie centrale de l'élément linéaire (110).

6. Ensemble de maille (10) selon l'une des revendications 2 à 5, si dépendante de la revendication 2, où les extrémités de l'élément de maille (100) et du noyau (200) sont raccordées par fusion thermique ou par collage.

7. Ensemble de maille (10, 40) selon l'une des revendications 1 à 6, comprenant en outre un tube d'insertion (400, 400A, 400B, 400C) s'étendant dans une première direction qui est une direction longitudinale, à l'intérieur duquel est formé un espace de canal (420), et prévu pour recevoir l'élément de maille (100) dans l'espace de canal. (420).

8. Ensemble de maille (10, 40) selon la revendication 7, où le tube d'insertion (400, 400A, 400B, 400C) comprend une partie (450) comprimée dans une deuxième direction perpendiculaire à la première direction, et où une section transversale de l'espace de canal (420) est de largeur réduite dans la deuxième direction dans la partie comprimée (450) et de largeur accrue dans une troisième direction perpendiculaire à la première direction et à la deuxième direction dans la partie comprimée (450).
